# EUROPEAN PATENT SPECIFICATION

(11) **EP 3 260 096 B1**
(45) Date of publication and mention of the grant of the patent: **01.08.2018**
(21) Application number: 16175476.7
(22) Date of filing: 21.06.2016
(51) Int. Cl.: A61F 13/42, A61F 13/84

(54) **WETNESS NOTIFICATION SYSTEM**
FEUCHTIGKEITSBENACHRICHTIGUNGSSYSTEM
SYSTÈME DE NOTIFICATION D'HUMIDITÉ

(43) Date of publication of application: 27.12.2017
(73) Proprietor: I-DING MEDICAL EQUIPMENT CO. LTD., Kaohsiung City 81352 (TW)
(72) Inventor: CHEN, Hung-Chi, Kaohsiung City 81352 (TW)
(74) Representative: Zimmermann, Tankred Klaus

(56) References cited:
- WO-A1-2006/118913
- CN-U- 203 252 811
- US-A1- 2004 207 530

## Description

The disclosure relates to a notification system, more particularly to a wetness notification system for detecting and notifying wetness in a clothing article.

A conventional diaper with a wetness sensing device includes a first non-woven fabric layer, two strip electrodes, a second non-woven fabric layer, a liquid absorbing layer made from a liquid absorbing material, a liquid-proof layer and two metallic sockets. A first side of the first non-woven fabric layer absorbs a liquid excreted from a human body, and the liquid permeates to a second side of the first non-woven fabric layer. The strip electrodes are attached onto the second side of the first non-woven fabric layer, and the strip electrodes are electrically connected when they come into contact with the liquid. A first side of the second non-woven fabric layer is attached to the second side of the first non-woven fabric layer and to the strip electrodes, and the strip electrodes are thus retained between the first and second non-woven fabric layers. The first side of the second non-woven fabric layer absorbs the liquid, which permeates to a second side of the second non-woven fabric layer. The liquid absorbing layer is attached to the second side of the second non-woven fabric layer, and absorbs that liquid permeated through the second non-woven fabric layer. The liquid-proof layer is made from a liquid proof material. A first side of the liquid-proof layer is attached to the second side of the liquid absorbing layer, and retains the liquid that permeated through the liquid absorbing layer to prevent the liquid from leaking out of the diaper. Each of the metallic sockets has a first engaging portion that engages with a respective one of two engaging bodies of an electrical detection device. This enables the electrical detection device to detect the electrical conduction between the strip electrodes when a liquid is excreted, and to send a wetness notification signal for notifying a caregiver to change the diaper according to the wetness notification signal.

However, the electrical detection device requires a battery for operation. Due to the absence of a low electricity notification mechanism for notification when the battery is low in electricity, untimely changing of the diaper may lead to skin allergies, rashes and other skin diseases. Moreover, the strip electrodes are sewn into the diaper, and sewing increases labor costs and may cause a rise in defective products.

Thus, a conventional wetness notification system for detecting wetness in a clothing article configured for absorbing urine has been developed to solve the problems mentioned above. However, such conventional system still lacks certain features, such as urine sampling and urinalysis, to provide early diagnosis for certain diseases. For example, by sampling a user's urine and analyzing the concentrations of the substances therein such as proteins, nitrite, glucose, ketones, bilirubin, occult blood, leukocytes, urobilinogen, etc., or analyzing the physical or chemical properties of the sampled urine such as specific gravity, pH value, etc., a more thorough understanding of the user's health status can be achieved.

Therefore, an object of the disclosure is to provide a wetness notification system that can alleviate at least one of the drawbacks of the prior art.

According to the disclosure, the wetness notification system includes a detecting unit, a clip unit, a receiver unit, and a sampling unit.

The detecting unit includes a sleeve body, a mounting seat combined with the sleeve body, a moisture detector mounted on the mounting seat and configured to output a notification signal when a surrounding moisture level detected thereby is greater than a threshold moisture level, a casing, a wireless transmitter disposed in the casing, a processor disposed in the casing, electrically connected to the moisture detector and the wireless transmitter, and configured to receive the notification signal outputted by the moisture detector and to transmit the notification signal through the wireless transmitter, and a battery disposed in the casing and configured to provide electricity to the moisture detector, the processor and the wireless transmitter.

The clip unit includes a first portion removably inserted into the sleeve body, a curved second portion extending from the first portion, and a third portion extending from the curved second portion, removably supporting the casing, and configured to cooperate with the first portion to clip the detecting unit onto the clothing article in a manner that the sleeve body and the mounting seat are disposed at an inner side of the clothing article and the third portion is disposed at an outer side of the clothing article.

The receiver unit includes a wireless receiver configured to communicate wirelessly with the wireless transmitter for receiving the notification signal therefrom, a notifying module, and a controller electrically connected to the wireless receiver and the notifying module and configured to drive the notifying module to output a wetness notification in response to receipt of the notification signal from the wireless receiver.

The sampling unit includes a substrate made of flexible material and removably attached to the mounting seat, and at least one sampling element disposed on the substrate for contacting and sampling urine within the clothing article for urinalysis.

Other features and advantages of the disclosure will become apparent in the following detailed description of the embodiments with reference to the accompanying drawings, of which:
Figure 1 is a perspective view illustrating an exemplary embodiment of a wetness notification system of the disclosure;
Figure 2 is an exploded perspective view illustrating a detecting unit and a clip unit of the exemplary embodiment shown in Figure 1;
Figure 3 is a schematic block diagram of the exemplary embodiment shown in Figure 1;
Figure 4 is a partially sectional view of the exemplary embodiment shown in Figure 1, illustrating that the wetness notification system is used with a clothing article; and
Figure 5 is an exploded perspective view illustrating a variation of the exemplary embodiment.

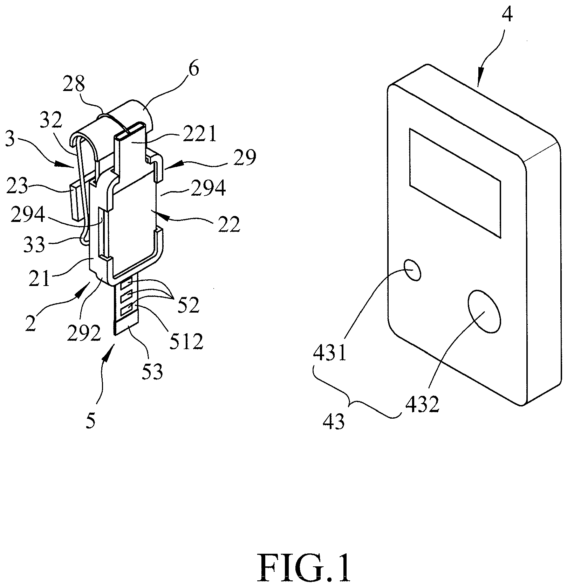

Referring to Figures 1 to 4, an exemplary embodiment of a wetness notification system for detecting and notifying wetness in a clothing article 7 according to the present disclosure includes a detecting unit 2, a clip unit 3, a receiver unit 4, a sampling unit 5, and a positioning member 6.

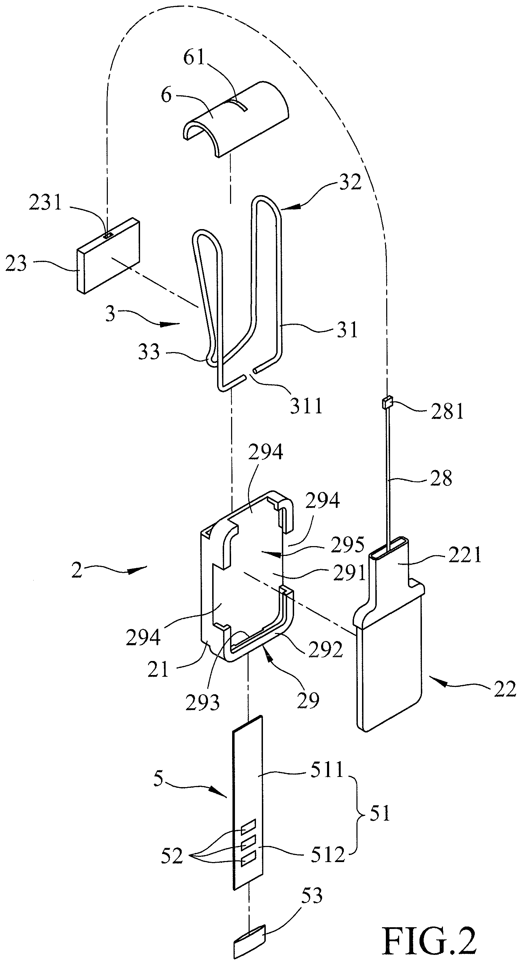

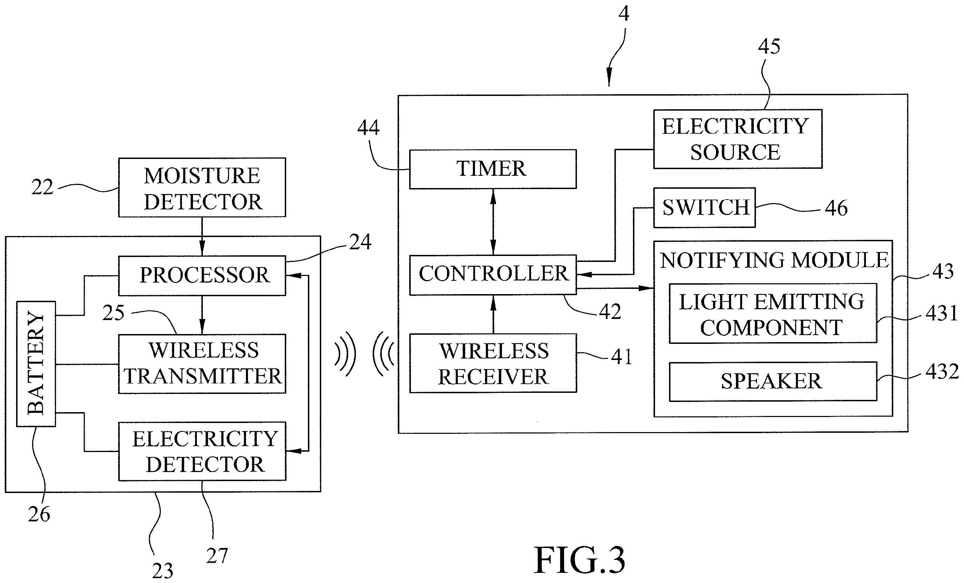

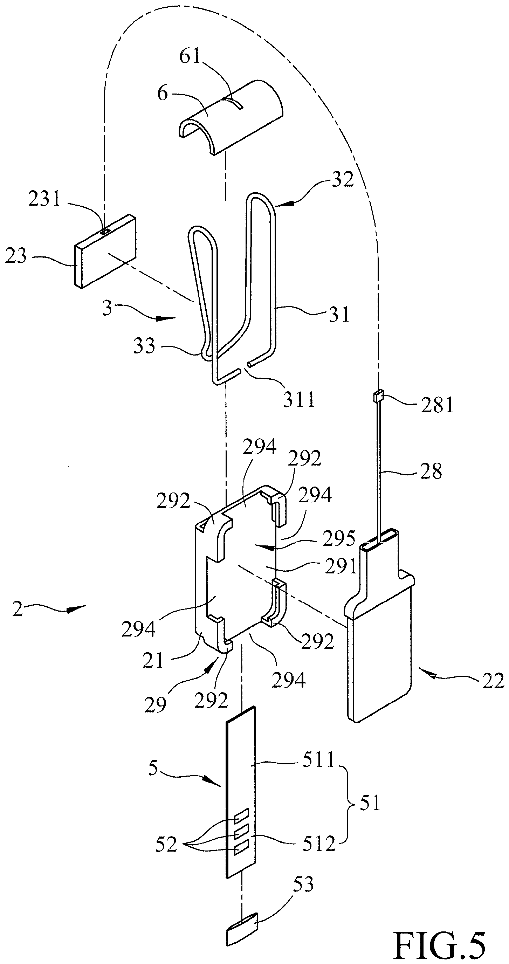

The detecting unit 2 includes a sleeve body 21, a mounting seat 29 combined with the sleeve body 21, a moisture detector 22 mounted on the mounting seat 29, a casing 23, a wireless transmitter 25 that is disposed in the casing 23, a processor 24 that is disposed in the casing 23 and that is electrically connected to the moisture detector 22 and the wireless transmitter 25, and a battery 26 that is disposed in the casing 23 and that provides electricity to the processor 24 and the wireless transmitter 25. In this embodiment, the detecting unit 2 further includes an electricity detector 27 that is disposed in the casing 23 and that is electrically connected to the battery 26 and the processor 24 (see Figure 3), and a transmission wire 28 as illustrated in Figure 2. As shown in Figure 2, the mounting seat 29 of this embodiment includes a base plate 291, and a positioning wall 292 that surrounds a periphery of the base plate 291, that cooperates with the base plate 291 to define an accommodating space 295 where the moisture detector 22 is tightly and removably disposed, and that is formed with a through hole 293 and a plurality of notches 294 (three notches are shown in Figure 2). The notches 294 are depressed toward the base plate 291 and facilitate deformation of the positioning wall 292 for removing the moisture detector 22 from the accommodating space 295. It is worth noting that, in certain embodiments, the positioning wall 292 may be formed with an additional notch instead of being formed with the through hole 293 as illustrated in Figure 5. In this embodiment, the casing 23 includes an input port 231 as illustrated in Figure 1. The battery 26 is electrically connected with the moisture detector 22, the wireless transmitter 25 and the electricity detector 27. The transmission wire 28 has a first end that is electrically connected to a connecting portion 221 of the moisture detector 22 which extends through one of the notches 294, and a second end that includes a connector 281 removably inserted into the input port 231 of the casing 23 so as to electrically connect the transmission wire 28 to the processor 24.

The clip unit 3 of this embodiment is a hollow frame made from a bent flexible loop-shaped member as illustrated in Figure 2, and has a first portion 31 removably inserted into the sleeve body 21, a curved second portion 32 that extends from the first portion 31, and a third portion 33 that extends from the curved second portion 32, that removably supports the casing 23, and that cooperates with the first portion 31 to clip the detecting unit 2 onto the clothing article 7. In this embodiment, the third portion 33 may have an overall width less than that of the first portion 31. The first portion 31 of this embodiment has two separate segments defining a gap 311 therebetween which facilitates deformation of the first portion 31 when the same is being removably inserted into the sleeve body 21.

The receiver unit 4 of this embodiment includes a wireless receiver 41 to communicate wirelessly with the wireless transmitter 25, a notifying module 43, a controller 42 electrically connected to the wireless receiver 41 and the notifying module 43, a timer 44 electrically coupled to the controller 42 and storing a predetermined period value, and an electricity source 45 electrically coupled with the controller 42 to provide electricity to the receiver unit 4. The controller 42 of this embodiment is configured to have a normal notification mode and a looping notification mode, which will be described in greater detail in the following paragraphs. The notifying module 43 may include a light emitting element 431 for emitting light, and a speaker 432 for outputting sound. It is worth noting that, in other embodiments, the timer 44 can be integrated into the controller 42, i.e., the controller 42 is capable of performing a time-counting function. A frequency used for wireless communication between the wireless transmitter 25 and the wireless receiver 41 can be preset in the factory. Alternatively, a tuner (not shown in the Figures) may be incorporated in the wetness notification system for adjusting the frequency in other embodiments of this disclosure.

As illustrated in Figure 2, the sampling unit 5 of this embodiment includes a substrate 51 that is made of a flexible material, such as silicone, and that is removably attached to the mounting seat 29, and a plurality of sampling elements 52 that are disposed on the substrate 51 for contacting and sampling urine within the clothing article 7. It should be noted that each sampling element 52 may be a test paper for simultaneously sampling and testing the urine like in this embodiment, or a vessel for sampling the urine used for subsequent urinalysis in other embodiments of this disclosure. As illustrated in Figures 1, 2 and 4, the substrate 51 includes a first segment 511 disposed in the accommodating space 295 and sandwiched in between the moisture detector 22 and the base plate 291, and a second segment 512 extending from the first segment 511 through the positioning wall 292 out of the mounting seat 29 and mounted with the sampling elements 52. In this embodiment, the second segment 512 extends from the first segment 511 through the through hole 293 formed on the positioning wall 292. In the embodiments where the through hole 293 is replaced by the additional notch 294, the second segment 512 may extend through such additional notch 294 as illustrated in Figure 5. In this embodiment, the sampling unit 5 further includes a protection sleeve 53 that is made of silicone, that covers a distal end of the second segment 512 without covering any of the sampling elements 52, and that has a curved end opposite to the first segment 511 for reducing discomfort of a user when the user is in direct contact therewith.

It is worth noting that the number of the sampling elements 52 included in the wetness notification system is not limited, i.e., to have one single sampling element 52 provided on the second segment 512 may suffice in other embodiments according to the present disclosure. Moreover, the configuration of the sampling elements 52 is not limited herein, for example, each of the sampling elements 52 maybe configured into a rectangular shape, a strip, etc. Furthermore, the sampling elements 52 may be removably attached onto the second segment 512 so that the sampling elements 52 may be easily replaced after use.

The positioning member 6 engages the clip unit 3 and is formed with a slit 61 for receiving a section of the transmission wire 28 so as to position the transmission wire 28 as illustrated in Figure 1.

As shown in Figures 1, 2 and 4, the wetness notification system can be applied on the clothing article 7 (such as pad-type or pant-type diapers), which is wearable on a human body 8. When in use, the first portion 31 of the clip unit 3 is inserted into the sleeve body 21, the casing 23 is disposed on the third portion 33 of the clip unit 3, and the transmission wire 28 is positioned by the slit 61 of the positioning member 6 with the connector 281 being plugged into the input port 231 of the casing 23. The sleeve body 21, the moisture detector 22, and the sampling unit 5 are all disposed at an inner side of the clothing article 7, preferably adjacent to the groin area of the human body 8, and the third portion 33 is disposed at an outer side of the clothing article 7. It may be noted that, in this embodiment, the clip unit 3 exhibits light-weight characteristics due to its hollow frame structure, and reduces the burden of the user when the user is carrying the clip unit 3. Moreover, the design of the curved second portion 32 can enhance the comfort of the user. Furthermore, the receiver unit 4 can be carried around by a caregiver or be placed at a specific location that can be seen by the caregiver.

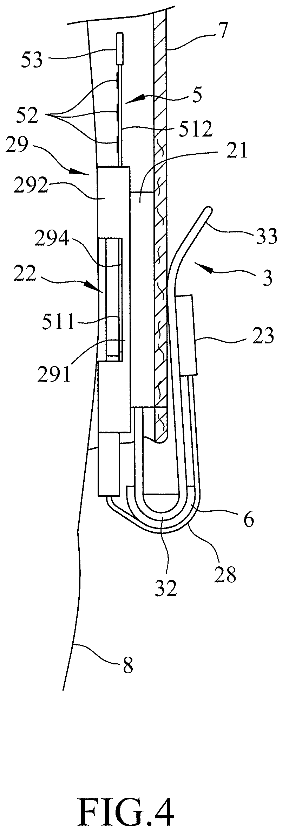

As shown in Figures 3 and 4, the moisture detector 22 is configured to output a notification signal when a surrounding moisture level detected thereby, e.g., the moisture around the groin area, is greater than a threshold moisture level upon the detecting unit 2 being clipped onto the clothing article 7. The processor 24 is configured to receive the notification signal outputted by the moisture detector 22, and to transmit the notification signal through the wireless transmitter 25. The wireless receiver 41 is configured to communicate wirelessly with the wireless transmitter 25 for receiving the notification signal therefrom, and the controller 42 is configured to receive the notification signal through the wireless receiver 41 of the receiver unit 4. When the controller 42 receives the notification signal for a first time via the wireless receiver 41, the controller 42 executes the normal notification mode to drive the notifying module 43 to output a wetness notification in response to receipt of the notification signal, and at the same time configures the timer 44 to start counting. The controller 42 configures the notifying module 43 to stop outputting the wetness notification when the timer 44 has counted to the predetermined period value. The wetness notification may be the light emitted by the light emitting element 431, and/or the sound outputted by the speaker 432, so as to alert the caregiver for replacement of the clothing article 7.

After the notifying module 43 stops outputting the wetness notification, if the controller 42 continues receiving the notification signal via the wireless receiver 41, the controller 42 executes the looping notification mode to configure the notifying module 43 to output the wetness notification intermittently until the controller 42 no longer receives the notification signal. The looping notification mode of the controller 42 helps to remind the caregiver to replace the clothing article 7 so as to prevent rashes and other skin diseases on the user.

Referring to Figures 3 and 4, when the clothing article 7 absorbs urine, the sampling elements 52 of the sampling unit 5 simultaneously contact the urine and perform the urine test, thus allowing the caregiver to observe the color on the sampling elements 52 while replacing the clothing article 7, and to compare the color with a color chart to get the results. Therefore, the caregiver can monitor the user's health status in real time and be able to watch for any possible diseases that may affect the user.

In this embodiment, the electricity detector 27 detects the residual electricity of the battery 26. When the residual electricity detected by the electricity detector 27 is lower than a threshold electricity value, the electricity detector 27 is configured to output a low battery signal to the processor 24, which is then configured to transmit the low battery signal through the wireless transmitter 25. When the controller 42 receives the low battery signal for a first time via the wireless receiver 41, the controller 42 executes the normal notification mode to drive the notifying module 43 to output a low electricity notification in response to receipt of the low battery signal, and at the same time configures the timer 44 to start counting.

The controller 42 configures the notifying module 43 to stop outputting the low electricity notification when the timer 44 has counted to the predetermined period value. The low electricity notification may be the light emitted by the light emitting element 431, and/or the sound outputted by the speaker 432. Similarly, after the notifying module 43 stops outputting the low electricity notification, if the controller 42 continues receiving the low battery signal via the wireless receiver 41 (such as when the battery 26 is yet to be replaced), the controller 42 executes the looping notification mode to configure the notifying module 43 to output the low electricity notification intermittently until the controller 42 no longer receives the low battery signal. In the same manner, when the residual electricity in the electricity source 45 is low, the controller 42 configures the notifying module 43 to output the low electricity notification until the electricity in the electricity source 45 is no longer low. The low electricity notification can prevent the caregiver from failing to replace the clothing article 7 when the notification module 43 cannot timely respond to the wetness in the clothing article 7 due to the lack of electricity in the battery 26 or in the electricity source 45.

In this embodiment, the moisture detector 22 and the clip unit 3 are removable from the mounting seat 29. The casing 23 that contains the processor 24, the wireless transmitter 25, the battery 26 and the electricity detector 27 is removable from the clip unit 3. The transmission wire 28 is also removable from the casing 23. Such configurations provide convenience in washing, repair and replacement of individual units in the wetness notification system.

Particularly, the receiver unit 4 further includes a switch 46 electrically coupled with the controller 42. When the controller 42 executes the normal notification mode to configure the notifying module 43 to output one of the wetness notification and the low electricity notification, the controller 42 configures the timer 44 to start counting, and the switch 46 may be operated by the caregiver to control the controller 42 to control in turn the notifying module 43 to stop outputting one of the wetness notification and the low electricity notification. When the timer 44 has counted to the predetermined period value and the controller 42 continues receiving the one of the notification signal and the low battery signal via the wireless receiver 41, the controller 42 executes the looped notification mode to configure the notifying module 43 to output the corresponding one of the wetness notification and the low electricity notification intermittently.

In summary, the inclusion of the sampling unit 5 in the wetness notification system of this disclosure not only allows for urinalysis to be performed immediately such that the caregivers can monitor the user's health status in real time, but can also notify the caregiver when the wetness notification system detects the wetness in the clothing article. Moreover, it is possible to use the urine sampled by the sampling elements 52 for bacterial culture. In addition, it is also possible to replace the sampling elements 52 if necessary. In addition to wetness detection by the moisture detector 22 and the processor 24, the electricity detector 27 detects the residual electricity of both the battery 26 and the electricity source 45, and the notifying module 43 outputs the notification signal (such as light and/or sound) more than once when low residual electricity is detected, such that the caregiver can replace the battery 26 or replenish the electricity in the electricity source 45. In such manner, the caregiver can replace the clothing article 7 to prevent rashes and other skin diseases on a wearer and be notified to keep the wetness notification system working. Moreover, the wetness notification system includes the moisture detector 22 and the clip unit 3 that are removable from the mounting seat 29, the casing 23 that is removable from the clip unit 3, and the transmission wire 28 that is removable from the casing 23, and such removal design provides convenience in washing, repair and replacement of individual units in the wetness notification system.

In the description above, for the purposes of explanation, numerous specific details have been set forth in order to provide a thorough understanding of the embodiment (s) . It will be apparent, however, to one skilled in the art, that one or more other embodiments may be practiced without some of these specific details. It should also be appreciated that reference throughout this specification to "one embodiment, " "an embodiment, " an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects.

## Claims

1. A wetness notification system for detecting and notifying wetness in a clothing article (7) that is configured for absorbing urine, the wetness notification system being **characterized by**:
a detecting unit (2) that includes
a sleeve body (21),
a mounting seat (29) combined with said sleeve body (21),
a moisture detector (22) mounted on said mounting seat (29), and configured to output a notification signal when a surrounding moisture level detected thereby is greater than a threshold moisture level,
a casing (23),
a wireless transmitter (25) disposed in said casing (23),
a processor (24) disposed in said casing (23), electrically connected to said moisture detector (22) and said wireless transmitter (25), and configured to receive the notification signal outputted by said moisture detector (22) and to transmit the notification signal through said wireless transmitter (25), and
a battery (26) disposed in said casing (23) and configured to provide electricity to said moisture detector (22), said processor (24) and said wireless transmitter (25);
a clip unit (3) that includes
a first portion (31) removably inserted into said sleeve body (21),
a curved second portion (32) extending from said first portion (31), and
a third portion (33) extending from said curved second portion (32), removably supporting said casing (23), and configured to cooperate with said first portion (31) to clip said detecting unit (2) onto the clothing article (7) in a manner that said sleeve body (21) and said mounting seat (29) are disposed at an inner side of the clothing article (7) and that said third portion (33) is disposed at an outer side of the clothing article (7);
a receiver unit (4) that includes a wireless receiver (41) configured to communicate wirelessly with said wireless transmitter (25) for receiving the notification signal therefrom, a notifying module (43), and a controller (42) electrically connected to said wireless receiver (41) and said notifying module (43) and configured to drive said notifying module (43) to output a wetness notification in response to receipt of the notification signal from said wireless receiver (41); and
a sampling unit (5) that includes a substrate (51) made of flexible material and removably attached to said mounting seat (29), and at least one sampling element (52) disposed on said substrate (51) for contacting and sampling urine within the clothing article (7) for urinalysis.

2. The wetness notification system as claimed in Claim 1, **characterized in that** said mounting seat (29) includes a base plate (291), and a positioning wall (292) surrounding a periphery of said base plate (291) and cooperating with said base plate (291) to define an accommodating space (295) where said moisture detector (22) is tightly disposed,
**characterized in that** said substrate (51) includes a first segment (511) disposed in the accommodating space (295) and sandwiched in between said moisture detector (22) and said base plate (291), and a second segment (512) extending from said first segment (511) through said positioning wall (292) out of said mounting seat (29) and mounted with said at least one sampling element (52) .

3. The wetness notification system as claimed in Claim 2, **characterized in that** said positioning wall (292) is formed with a through hole (293), and said second segment (512) extends through said through hole (293).

4. The wetness notification system as claimed in Claim 2, **characterized in that** said positioning wall (292) is formed with at least one notch (294) depressed toward said base plate (291) and facilitating deformation of said positioning wall (292) for removing said moisture detector (22) from the accommodating space (295).

5. The wetness notification system as claimed in Claim 4, **characterized in that** said second segment (512) extends through said at least one notch (294).

6. The wetness notification system as claimed in Claim 2, **characterized in that** said positioning wall (292) is formed with a plurality of notches (294) depressed toward said base plate (291), and said second segment (512) extends through one of said notches (294).

7. The wetness notification system as claimed in Claim 2, **characterized in that** said sampling unit (5) includes a plurality of said sampling elements (52) disposed on said second segment (512) of said substrate (51).

8. The wetness notification system as claimed in Claim 2, **characterized in that** said sampling unit (5) further includes a protection sleeve (53) that is made of silicone, that covers a distal end of said second segment (512) without covering said at least one sampling element (52), and that has a curved end opposite to said first segment (511).

9. The wetness notification system as claimed in Claim 1, **characterized in that** said mounting seat (29) includes:
a base plate (291); and
a positioning wall (292) surrounding a periphery of said base plate (291), cooperating with said base plate (291) to define an accommodating space (295) where said moisture detector (22) is tightly disposed, and being formed with a through hole (293) and a plurality of notches (294) that are depressed toward said base plate (291) and that facilitate deformation of said positioning wall (292) for removing said moisture detector (22) from the accommodating space (295),
**characterized in that** said substrate (51) includes a first segment (511) disposed in the accommodating space (295) and sandwiched in between said moisture detector (22) and said base plate (291), and a second segment (512) extending from said first segment (511) through said through hole (293) out of said mounting seat (29) and mounted with said at least one sampling element (52),
**characterized in that** said sampling unit (5) further includes a protection sleeve (53) that is made of silicone, that covers a distal end of said second segment (512) without covering said at least one sampling element (52), and that has a curved end opposite to said first segment (511).

10. The wetness notification system as claimed in Claim 1, **characterized in that** said mounting seat (29) includes:
a base plate (291); and
a positioning wall (292) surrounding a periphery of said base plate (291), cooperating with said base plate (291) to define an accommodating space (295) where said moisture detector (22) is tightly disposed, and being formed with a plurality of notches (294) that are depressed toward said base plate (291) and that facilitate deformation of said positioning wall (292) for removing said moisture detector (22) from the accommodating space (295),
**characterized in that** said substrate (51) includes a first segment (511) disposed in the accommodating space (295) and sandwiched in between said moisture detector (22) and said base plate (291), and a second segment (512) extending from said first segment (511) through one of said notches (294) out of said mounting seat (29) and being mounted with said at least one sampling element (52), and
**characterized in that** said sampling unit (5) further includes a protection sleeve (53) that is made of silicone, that covers a distal end of said second segment (512) without covering said at least one sampling element (52), and that has a curved end opposite to said first segment (511).

11. The wetness notification system as claimed in Claim 1, **characterized in that** said detecting unit (2) further includes an electricity detector (27) disposed in said casing (23), electrically connected to said battery (26) and said processor (24), and configured to detect residual electricity stored in said battery (26) and to output a low battery signal to said processor (24) when the residual electricity detected thereby is lower than a threshold electricity value,
**characterized in that** said processor (24) is further configured to transmit the low battery signal through said wireless transmitter (25), and
**characterized in that** said wireless receiver (41) is configured to receive the low battery signal from said wireless transmitter (25), and said controller (42) is further configured to drive said notifying module (43) to output a low electricity notification in response to receipt of the low battery signal from said wireless receiver (41).

12. The wetness notification system as claimed in Claim 1, **characterized in that** said detecting unit (2) further includes a transmission wire (28) having a first end electrically connected with said moisture detector (22), and a second end electrically connected to said processor (24) .

13. The wetness notification system as claimed in Claim 12, **characterized in that** said casing (23) includes an input port (231), and said second end of said transmission wire (28) includes a connector (281) removably inserted into said input port (231).

14. The wetness notification system as claimed in Claim 12, further **characterized by** a positioning member (6) that engages said clip unit (3) and that is formed with a slit (61) for receiving a section of said transmission wire (28) so as to position said transmission wire (28).

15. The wetness notification system as claimed in Claim 1, **characterized in that** said at least one sampling element (52) is one of a test paper for sampling and testing the urine at the same time and a vessel for sampling the urine used for the urinalysis.

## Patentansprüche

1. Ein Nässemeldesystem zum Erfassen und Melden von Nässe in einem Bekleidungsartikel (7), der dazu konfiguriert ist, Urin zu absorbieren, wobei das Nässemeldesystem durch folgende Merkmale gekennzeichnet ist:
eine Erfassungseinheit (2), die Folgendes umfasst:
einen Einfassungskörper (21),
einen Anbringsitz (29), der mit dem Einfassungskörper (21) kombiniert ist,
einen Feuchtigkeitsdetektor (22), der an dem Anbringsitz (29) angebracht und dazu konfiguriert ist, ein Meldesignal auszugeben, wenn ein durch denselben erfasster Umgebungsfeuchtigskeitspegel höher ist als ein Schwellenfeuchtigkeitspegel,
ein Gehäuse (23),
einen in dem Gehäuse (23) angeordneten drahtlosen Sender (25),
einen in dem Gehäuse (23) angeordneten Prozessor (24), der elektrisch mit dem Feuchtigkeitsdetektor (22) und dem drahtlosen Sender (25) verbunden und dazu konfiguriert ist, dass durch den Feuchtigkeitsdetektor (22) ausgegebene Meldesignal zu empfangen und das Meldesignal durch den drahtlosen Sender (25) zu senden, und
eine in dem Gehäuse (23) angeordnete Batterie (26), die dazu konfiguriert ist, den Feuchtigkeitsdetektor (22), den Prozessor (24) und den drahtlosen Sender (25) mit Elektrizität zu versorgen;
eine Klemmeinheit (3), die folgende Merkmale umfasst:
einen ersten Abschnitt (31), der herausnehmbar in den Einfassungskörper (21) eingefügt ist,
einen gekrümmten zweiten Abschnitt (32), der sich von dem ersten Abschnitt (31) aus erstreckt, und
einen dritten Abschnitt (33), der sich von dem gekrümmten zweiten Abschnitt (32) aus erstreckt und das Gehäuse (23) auf abnehmbare Weise trägt und dazu konfiguriert ist, mit dem ersten Abschnitt (31) dahin gehend zusammenzuwirken, die Erfassungseinheit (2) derart auf den Bekleidungsartikel (7) zu klemmen, dass der Einfassungskörper (21) und der Anbringsitz (29) auf einer Innenseite des Bekleidungsartikels (7) angeordnet sind und dass der dritte Abschnitt (33) auf einer Außenseite des Bekleidungsartikels (7) angeordnet ist;
eine Empfängereinheit (4), die einen drahtlosen Empfänger (41), der dazu konfiguriert ist, drahtlos mit dem drahtlosen Sender (25) zu kommunizieren, um das Meldesignal von demselben zu empfangen, ein Meldemodul (43) und eine Steuerung (42), die mit dem drahtlosen Empfänger (41) und dem Meldemodul (43) elektrisch verbunden und dazu konfiguriert ist, das Meldemodul (43) dahin gehend anzusteuern, ansprechend auf einen Empfang des Meldesignals von dem drahtlosen Empfänger (41) hin eine Nässemeldung auszugeben, umfasst; und
eine Probenahmeeinheit (5), die ein Substrat (51), das aus flexiblem Material hergestellt ist und abnehmbar an dem Anbringsitz (29) befestigt ist, und zumindest ein Probenahmeelement (52), das an dem Substrat (51) angeordnet ist, um Urin in dem Bekleidungsartikel (7) zu kontaktieren und zum Zweck einer Urinanalyse eine Probe davon zu nehmen, umfasst.

2. Das Nässemeldesystem gemäß Anspruch 1, **dadurch gekennzeichnet, dass** der Anbringsitz (29) eine Basisplatte (291) und eine Positionierungswand (292), die eine Peripherie der Basisplatte (291) umgibt und mit der Basisplatte (291) dahin gehend zusammenwirkt, einen Unterbringungsraum (295) zu definieren, wo der Feuchtigkeitsdetektor (22) fest angeordnet ist, umfasst,
**dadurch gekennzeichnet, dass** das Substrat (51) ein erstes Segment (511), das in dem Unterbringungsraum (295) angeordnet und zwischen dem Feuchtigkeitsdetektor (22) und der Basisplatte (291) platziert ist, und ein zweites Segment (512), das sich von dem ersten Segment (511) durch die Positionierungswand (292) hindurch und aus dem Anbringsitz (29) heraus erstreckt und an dem zumindest einen Probenahmeelement (52) angebracht ist, umfasst.

3. Das Nässemeldesystem gemäß Anspruch 2, **dadurch gekennzeichnet, dass** die Positionierungswand (292) mit einem Durchgangsloch (293) gebildet ist und sich das zweite Segment (512) durch das Durchgangsloch (293) hindurch erstreckt.

4. Das Nässemeldesystem gemäß Anspruch 2, **dadurch gekennzeichnet, dass** die Positionierungswand (292) mit zumindest einer Kerbe (294) gebildet ist, die zu der Basisplatte (291) hin vertieft ist und eine Verformung der Positionierungswand (292) ermöglicht, um den Feuchtigkeitsdetektor (22) aus dem Unterbringungsraum (295) zu entfernen.

5. Das Nässemeldesystem gemäß Anspruch 4, **dadurch gekennzeichnet, dass** sich das zweite Segment (512) durch die zumindest eine Kerbe (294) hindurch erstreckt.

6. Das Nässemeldesystem gemäß Anspruch 2, **dadurch gekennzeichnet, dass** die Positionierungswand (292) mit einer Mehrzahl von Kerben (294) gebildet ist, die zu der Basisplatte (291) hin vertieft sind, und sich das zweite Segment (512) durch eine der Kerben (294) hindurch erstreckt.

7. Das Nässemeldesystem gemäß Anspruch 2, **dadurch gekennzeichnet, dass** die Probenahmeeinheit (5) eine Mehrzahl der Probenahmeelemente (52) umfasst, die an dem zweiten Segment (512) des Substrats (51) angeordnet sind.

8. Das Nässemeldesystem gemäß Anspruch 2, **dadurch gekennzeichnet, dass** die Probenahmeeinheit (5) ferner eine Schutzeinfassung (53) umfasst, die aus Silikon hergestellt ist, die ein distales Ende des zweiten Segments (512) abdeckt, ohne das zumindest eine Probenahmeelement (52) zu bedecken, und die ein gekrümmtes Ende aufweist, das dem ersten Segment (511) gegenüberliegt.

9. Das Nässemeldesystem gemäß Anspruch 1, **dadurch gekennzeichnet, dass** der Anbringsitz (29) folgende Merkmale umfasst:
eine Basisplatte (291); und
eine Positionierungswand (292), die eine Peripherie der Basisplatte (291) umgibt und mit der Basisplatte (291) dahin gehend zusammenwirkt, einen Unterbringungsraum (295) zu definieren, wo der Feuchtigkeitsdetektor (22) fest angeordnet ist, und die mit einem Durchgangsloch (293) und einer Mehrzahl von Kerben (294) gebildet ist, die zu der Basisplatte (291) hin vertieft sind und eine Verformung der Positionierungswand (292) ermöglichen, um den Feuchtigkeitsdetektor (22) aus dem Unterbringungsraum (295) zu entfernen,
**dadurch gekennzeichnet, dass** das Substrat (51) ein erstes Segment (511), das in dem Unterbringungsraum (295) angeordnet und zwischen dem Feuchtigkeitsdetektor (22) und der Basisplatte (291) platziert ist, und ein zweites Segment (512), das sich von dem ersten Segment (511) durch das Durchgangsloch (293) hindurch und aus dem Anbringsitz (29) heraus erstreckt und an dem zumindest einen Probenahmeelement (52) angebracht ist, umfasst,
**dadurch gekennzeichnet, dass** die Probenahmeeinheit (5) ferner eine Schutzeinfassung (53) umfasst, die aus Silikon hergestellt ist, die ein distales Ende des zweiten Segments (512) abdeckt, ohne das zumindest eine Probenahmeelement (52) zu bedecken, und die ein gekrümmtes Ende aufweist, das dem ersten Segment (511) gegenüberliegt.

10. Das Nässemeldesystem gemäß Anspruch 1, **dadurch gekennzeichnet, dass** der Anbringsitz (29) folgende Merkmale umfasst:
eine Basisplatte (291); und
eine Positionierungswand (292), die eine Peripherie der Basisplatte (291) umgibt und mit der Basisplatte (291) dahin gehend zusammenwirkt, einen Unterbringungsraum (295) zu definieren, wo der Feuchtigkeitsdetektor (22) fest angeordnet ist, und die mit einer Mehrzahl von Kerben (294) gebildet ist, die zu der Basisplatte (291) hin vertieft sind und eine Verformung der Positionierungswand (292) ermöglichen, um den Feuchtigkeitsdetektor (22) aus dem Unterbringungsraum (295) zu entfernen,
**dadurch gekennzeichnet, dass** das Substrat (51) ein erstes Segment (511), das in dem Unterbringungsraum (295) angeordnet und zwischen dem Feuchtigkeitsdetektor (22) und der Basisplatte (291) platziert ist, und ein zweites Segment (512), das sich von dem ersten Segment (511) durch eine der Kerben (294) hindurch und aus dem Anbringsitz (29) heraus erstreckt und an dem zumindest einen Probenahmeelement (52) angebracht ist, umfasst, und
**dadurch gekennzeichnet, dass** die Probenahmeeinheit (5) ferner eine Schutzeinfassung (53) umfasst, die aus Silikon hergestellt ist, die ein distales Ende des zweiten Segments (512) abdeckt, ohne das zumindest eine Probenahmeelement (52) zu bedecken, und die ein gekrümmtes Ende aufweist, das dem ersten Segment (511) gegenüberliegt.

11. Das Nässemeldesystem gemäß Anspruch 1, **dadurch gekennzeichnet, dass** die Erfassungseinheit (2) ferner einen Elektrizitätsdetektor (27) umfasst, der in dem Gehäuse (23) angeordnet ist, mit der Batterie (26) und dem Prozessor (24) elektrisch verbunden ist und dazu konfiguriert ist, eine in der Batterie (26) gespeicherte Restelektrizität zu erfassen und an den Prozessor (24) ein Batterie-Schwach-Signal auszugeben, wenn die durch ihn erfasste Restelektrizität niedriger ist als ein Schwellenelektrizitätswert,
**dadurch gekennzeichnet, dass** der Prozessor (24) ferner dazu konfiguriert ist, das Batterie-Schwach-Signal durch den drahtlosen Sender (25) zu senden, und
**dadurch gekennzeichnet, dass** der drahtlose Empfänger (41) dazu konfiguriert ist, das Batterie-Schwach-Signal von dem drahtlosen Sender (25) zu empfangen, und die Steuerung (42) ferner dazu konfiguriert ist, das Meldemodul (43) dahin gehend anzusteuern, ansprechend auf einen Empfang des Batterie-Schwach-Signals von dem drahtlosen Empfänger (41) hin eine Elektrizität-Schwach-Meldung auszugeben.

12. Das Nässemeldesystem gemäß Anspruch 1, **dadurch gekennzeichnet, dass** die Erfassungseinheit (2) ferner einen Sendedraht (28) umfasst, der ein mit dem Feuchtigkeitsdetektor (22) elektrisch verbundenes erstes Ende und ein mit dem Prozessor (24) elektrisch verbundenes zweites Ende aufweist.

13. Das Nässemeldesystem gemäß Anspruch 12, **dadurch gekennzeichnet, dass** das Gehäuse (23) eine Eingangsöffnung (231) umfasst und das zweite Ende des Sendedrahtes (28) einen Verbinder (281) umfasst, der herausnehmbar in die Eingangsöffnung (231) eingefügt ist.

14. Das Nässemeldesystem gemäß Anspruch 12, das ferner durch ein Positionierungsbauglied (6) gekennzeichnet ist, das die Klemmeinheit (3) in Eingriff nimmt und das mit einem Schlitz (61) zum Aufnehmen eines Teils des Sendedrahtes (28), um den Sendedraht (28) zu positionieren, gebildet ist.

15. Das Nässemeldesystem gemäß Anspruch 1, **dadurch gekennzeichnet, dass** das zumindest eine Probenahmeelement (52) entweder ein Testpapier zum gleichzeitigen Probenehmen und Testen des Urins oder ein Gefäß zur Probenahme des für die Urinanalyse verwendeten Urins ist.

## Revendications

1. Système de notification d'humidité pour détecter et notifier de l'humidité dans un article vestimentaire (7) qui est configuré pour absorber l'urine, le système de notification d'humidité étant **caractérisé par**:
une unité de détection (2) qui comporte
un corps de douille (21),
un siège de montage (29) combiné avec ledit corps de douille (21),
un détecteur d'humidité (22) monté sur ledit siège de montage (29) et configuré pour sortir un signal de notification lorsqu'un niveau d'humidité environnant détecté par ce dernier est supérieur à un niveau d'humidité de seuil,
un boîtier (23),
un émetteur sans fil (25) disposé dans ledit boîtier (23),
un processeur (24) disposé dans ledit boîtier (23), connecté électriquement audit détecteur d'humidité (22) et audit émetteur sans fil (25), et configuré pour recevoir le signal de notification sorti par ledit détecteur d'humidité (22) et pour transmettre le signal de notification par l'intermédiaire dudit émetteur sans fil (25), et
une batterie (26) disposée dans ledit boîtier (23) et configurée pour fournir de l'électricité audit détecteur d'humidité (22), audit processeur (24) et audit émetteur sans fil (25);
une unité de clip (3) qui comporte
une première partie (31) insérée de manière amovible dans ledit corps de douille (21),
une deuxième partie courbe (32) s'étendant à partir de ladite première partie (31), et
une troisième partie (33) s'étendant à partir de ladite deuxième partie courbe (32), supportant de manière amovible ledit boîtier (23), et configurée pour coopérer avec ladite première partie (31) pour attacher ladite unité de détection (2) à l'article vestimentaire (7) de manière que ledit corps de douille (21) et ledit siège de montage (29) soient disposés d'un côté intérieur de l'article vestimentaire (7) et que ladite troisième partie (33) soit disposée d'un côté extérieur de l'article vestimentaire (7);
une unité de réception (4) qui comporte un récepteur sans fil (41) configuré pour communiquer sans fil avec ledit émetteur sans fil (25) pour recevoir le signal de notification de ce dernier, un module de notification (43) et une commande (42) connectée électriquement audit récepteur sans fil (41) et audit module de notification (43) et configurée pour commander ledit module de notification (43) pour sortir une notification d'humidité en réponse à la réception du signal de notification dudit récepteur sans fil (41); et
une unité d'échantillonnage (5) qui comporte un substrat (51) réalisé en un matériau flexible et fixé de manière amovible audit siège de montage (29), et au moins un élément d'échantillonnage (52) disposé sur ledit substrat (51) pour entrer en contact avec et échantillonner l'urine dans l'article vestimentaire (7) pour l'analyse d'urine.

2. Système de notification d'humidité selon la revendication 1, **caractérisé par le fait que** ledit siège de montage (29) comporte une plaque de base (291) et une paroi de positionnement (292) entourant un pourtour de ladite plaque de base (291) et coopérant avec ladite plaque de base (291) pour définir un espace de logement (295) où est disposé de manière serrée ledit détecteur d'humidité (22),
**caractérisé par le fait que** ledit substrat (51) comporte un premier segment (511) disposé dans l'espace de logement (295) et pris en sandwich entre ledit détecteur d'humidité (22) et ladite plaque de base (291), et un deuxième segment (512) s'étendant dudit premier segment (511) à travers ladite paroi de positionnement (292) hors dudit siège de montage (29) et monté avec ledit au moins un élément d'échantillonnage (52).

3. Système de notification d'humidité selon la revendication 2, **caractérisé par le fait que** ladite paroi de positionnement (292) est formée avec un trou traversant (293), et ledit deuxième segment (512) s'étend à travers ledit trou traversant (293).

4. Système de notification d'humidité selon la revendication 2, **caractérisé par le fait que** ladite paroi de positionnement (292) est formée avec au moins une encoche (294) enfoncée vers ladite plaque de base (291) et facilitant la déformation de ladite paroi de positionnement (292) pour retirer ledit détecteur d'humidité (22) de l'espace de logement (295).

5. Système de notification d'humidité selon la revendication 4, **caractérisé par le fait que** ledit deuxième segment (512) s'étend à travers ladite au moins une encoche (294).

6. Système de notification d'humidité selon la revendication 2, **caractérisé par le fait que** ladite paroi de positionnement (292) est formée avec une pluralité d'encoches (294) enfoncées vers ladite plaque de base (291), et ledit deuxième segment (512) s'étend à travers l'une desdites encoches (294).

7. Système de notification d'humidité selon la revendication 2, **caractérisé par le fait que** ladite unité d'échantillonnage (5) comporte une pluralité desdits éléments d'échantillonnage (52) disposés sur ledit deuxième segment (512) dudit substrat (51).

8. Système de notification d'humidité selon la revendication 2, **caractérisé par le fait que** ladite unité d'échantillonnage (5) comporte par ailleurs un manchon de protection (53) qui est réalisé en silicone, qui recouvre une extrémité distale dudit deuxième segment (512) sans recouvrir ledit au moins un élément d'échantillonnage (52), et qui présente une extrémité courbe opposée audit premier segment (511).

9. Système de notification d'humidité selon la revendication 1, **caractérisé par le fait que** ledit siège de montage (29) comporte:
une plaque de base (291); et
une paroi de positionnement (292) entourant un pourtour de ladite plaque de base (291), coopérant avec ladite plaque de base (291) pour définir un espace de logement (295) où est disposé de manière serrée ledit détecteur d'humidité (22), et formée avec un trou traversant (293) et une pluralité d'encoches (294) qui sont enfoncées vers ladite plaque de base (291) et qui facilitent la déformation de ladite paroi de positionnement (292) pour retirer ledit détecteur d'humidité (22) de l'espace de logement (295),
**caractérisé par le fait que** ledit substrat (51) comporte un premier segment (511) disposé dans l'espace de logement (295) et pris en sandwich entre ledit détecteur d'humidité (22) et ladite plaque de base (291), et un deuxième segment (512) s'étendant à partir dudit premier segment (511) à travers ledit trou traversant (293) hors dudit siège de montage (29) et monté avec ledit au moins un élément d'échantillonnage (52),
**caractérisé par le fait que** ladite unité d'échantillonnage (5) comporte par ailleurs un manchon de protection (53) qui est réalisé en silicone, qui recouvre une extrémité distale dudit deuxième segment (512) sans recouvrir ledit au moins un élément d'échantillonnage (52), et qui présente une extrémité courbe opposée audit premier segment (511).

10. Système de notification d'humidité selon la revendication 1, **caractérisé par le fait que** ledit siège de montage (29) comporte:
une plaque de base (291); et
une paroi de positionnement (292) entourant un pourtour de ladite plaque de base (291), coopérant avec ladite plaque de base (291) pour définir un espace de logement (295) où est disposé de manière serrée ledit détecteur d'humidité (22), et formée avec une pluralité d'encoches (294) qui sont enfoncées vers ladite plaque de base (291) et qui facilitent la déformation de ladite paroi de positionnement (292) pour retirer ledit détecteur d'humidité (22) de l'espace de logement (295),
**caractérisé par le fait que** ledit substrat (51) comporte un premier segment (511) disposé dans l'espace de logement (295) et pris en sandwich entre ledit détecteur d'humidité (22) et ladite plaque de base (291), et un deuxième segment (512) s'étendant à partir dudit premier segment (511) à travers l'une desdites encoches (294) hors dudit siège de montage (29) et monté avec ledit au moins un élément d'échantillonnage (52), et
**caractérisé par le fait que** ladite unité d'échantillonnage (5) comporte par ailleurs un manchon de protection (53) réalisé en silicone, qui recouvre une extrémité distale dudit deuxième segment (512) sans recouvrir ledit au moins un élément d'échantillonnage (52), et qui présente une extrémité courbe opposée audit premier segment (511).

11. Système de notification d'humidité selon la revendication 1, **caractérisé par le fait que** ladite unité de détection (2) comporte par ailleurs un détecteur d'électricité (27) disposé dans ledit boîtier (23), connecté électriquement à ladite batterie (26) et audit processeur (24), et configuré pour détecter l'électricité résiduelle accumulée dans ladite batterie (26) et pour sortir un faible signal de batterie vers ledit processeur (24) lorsque l'électricité résiduelle détectée est ainsi inférieure à une valeur d'électricité de seuil,
**caractérisé par le fait que** ledit processeur (24) est par ailleurs configuré pour transmettre le faible signal de batterie par l'intermédiaire dudit émetteur sans fil (25), et
**caractérisé par le fait que** ledit récepteur sans fil (41) est configuré pour recevoir le faible signal de batterie dudit émetteur sans fil (25), et ladite commande (42) est par ailleurs configurée pour commander ledit module de notification (43) pour sortir une notification de faible électricité en réponse à la réception du signal de batterie basse dudit récepteur sans fil (41).

12. Système de notification d'humidité selon la revendication 1, **caractérisé par le fait que** ladite unité de détection (2) comporte par ailleurs un fil de transmission (28) présentant une première extrémité connectée électriquement audit détecteur d'humidité (22), et une deuxième extrémité connectée électriquement audit processeur (24).

13. Système de notification d'humidité selon la revendication 12, **caractérisé par le fait que** ledit boîtier (23) comporte un orifice d'entrée (231), et ladite deuxième extrémité dudit câble de transmission (28) comporte un connecteur (281) inséré de manière amovible dans ledit orifice d'entrée (231).

14. Système de notification d'humidité selon la revendication 12, **caractérisé par** ailleurs par un élément de positionnement (6) qui vient en prise avec ladite unité de clip (3) et qui est formé avec une fente (61) destinée à recevoir un segment dudit fil de transmission (28) de manière à positionner ledit fil de transmission (28).

15. Système de notification d'humidité selon la revendication 1, **caractérisé par le fait que** ledit au moins un élément d'échantillonnage (52) est l'un parmi un papier de test destiné à échantillonner et tester l'urine en même temps et un récipient pour échantillonner l'urine utilisée pour l'analyse d'urine.
